# FASCICULE DE BREVET EUROPEEN

(11) **EP 0 637 740 B1**
(45) Date de publication et mention de la délivrance du brevet: **03.03.1999**
(21) Numéro de dépôt: 94401796.1
(22) Date de dépôt: 03.08.1994
(51) Int. Cl.: G01N 1/10, G21F 7/06

(54) **Dispositif de remplissage d'un récipient clos par une aiguille, muni de moyens de nettoyage**
Vorrichtung mit Reinigungsmittel zum Füllen von geschlossenen Behältern mit einer Nadel
Device with cleaning means for filling a closed container with a needle

(30) Priorité: 05.08.1993 FR 9309661
(43) Date de publication de la demande: 08.02.1995
(73) Titulaire: COMPAGNIE GENERALE DES MATIERES NUCLEAIRES (COGEMA), 78141 Vélizy-Villacoublay (FR)
(72) Inventeur: Herbreteau, Michel, F-50100 Cherbourg (FR); Marchand, Robert, F-50120 Equeurdreville-Hainneville (FR); Delahaye, Arnaud, F-50110 Tourlaville (FR)
(74) Mandataire: Ilgart, Jean-Christophe

(56) Documents cités:
- EP-A- 0 063 971
- EP-A- 0 078 212
- EP-A- 0 472 831
- EP-A- 0 511 097
- EP-A- 0 522 959
- DE-A- 3 044 424

## Description

L'invention concerne un dispositif de remplissage d'un récipient clos par une aiguille, muni de moyens de nettoyage.

Elle trouve notamment emploi dans une installation d'analyse de liquides dangereux, notamment radioactifs, où les récipients contenant les échantillons de liquide sont transportés par voie pneumatique dans un réseau de conduites entre différentes stations et en particulier des stations d'analyse. Il est cependant nécessaire de remplir au préalable les récipients, et c'est pourquoi une de ces stations du réseau est consacrée au remplissage. Cette station comporte essentiellement au moins un élévateur de liquide de remplissage terminé par une aiguille de perçage du récipient et un fourreau comprenant un fond percé devant l'aiguille. Le récipient à remplir est posé dans une nacelle coulissant dans le fourreau, qui reste suspendue à quelque distance du fond du fourreau pendant la plus grande partie des opérations de remplissage et qui est abaissée au fond du fourreau pour provoquer le perçage du fond du récipient par l'aiguille. Un tel dispositif est connu du document EP-A-0 511 097.

Les inconvénients qui résultent de cette conception sont liés à la pollution de l'enveloppe du récipient, voire du fond du fourreau. L'élévateur de liquide de remplissage peut en effet être sujet à des défauts de fonctionnement qui se traduisent par de petits jaillissements de liquide qui éclaboussent le récipient et le fond du fourreau, ou de simples suintements qui coulent le long de l'aiguille et se déposent sur le fond du récipient autour du trou de perçage. La grande vitesse (15 mètres à la seconde) acquise par les récipients pendant leur transport dans les canalisations provoque alors généralement une dispersion des gouttelettes dans les conduits et une pollution susceptible de se propager dans tout le réseau à cause notamment des mouvements de l'air de déplacement .

L'invention est relative à un perfectionnement d'une installation de remplissage existante et a pour objet de nettoyer le fond du récipient et la face interne de la paroi de fond du fourreau pour les débarrasser des liquides polluants qui risquent de les souiller.

Elle concerne sous sa forme la plus générale un dispositif de remplissage d'un récipient clos, comprenant : un élévateur de liquide de remplissage terminé par une aiguille de perçage d'un fond du récipient ; un fourreau ayant un fond constitué par une paroi percée devant l'aiguille, mobile vers l'aiguille et se raccordant à un réseau de conduits de transport du récipient par voie pneumatique ; et une nacelle située dans le fourreau et agencée pour suspendre le récipient un peu au-dessus du fond du fourreau ; caractérisé en ce que le fourreau est équipé de buses d'aspersion de liquide de lavage vers le fond du récipient suspendu dans la nacelle et vers la face interne de la paroi du fond du fourreau.

Le liquide polluant est alors amené à ruisseler avec le liquide de lavage et à couler au-dessous du fourreau et de l'aiguille jusque dans un égout où il ne peut plus nuire.

D'autres moyens peuvent compléter l'invention, tels que des buses supplémentaires d'aspersion du liquide de lavage vers l'aiguille et des moyens de séchage du fond du récipient et de la face interne de la paroi du fond du fourreau. De plus, il est avantageux que les buses soient orientées de façon à asperger le fond du récipient obliquement et à produire un rebondissement du liquide de lavage du fond du récipient vers la face interne du fond du fourreau.

L'invention va maintenant être décrite au moyen des figures suivantes :
- la figure 1 est une vue générale du dispositif de remplissage,
- la figure 2 est un détail montrant les parties essentielles,
- la figure 3 est une coupe sur la ligne III-III de la figure 2,
- et la figure 4 est un détail de la figure 3 illustrant le mode de lavage.

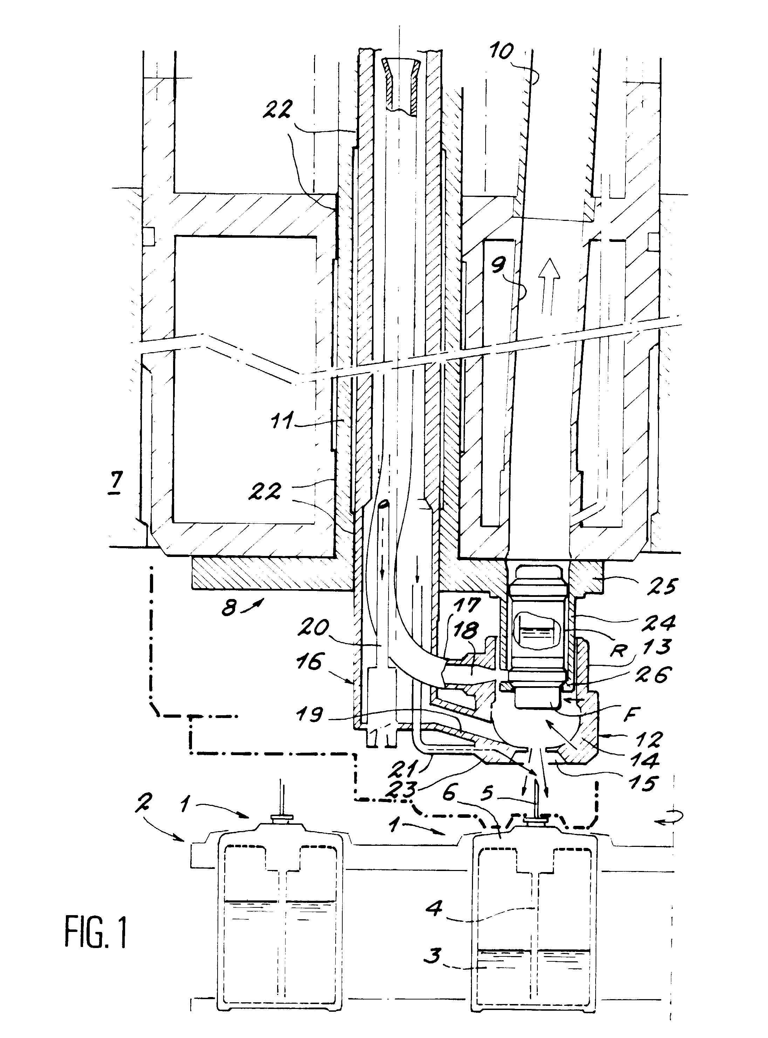

L'installation de remplissage comprend en réalité (figure 1) plusieurs têtes de remplissage 1 disposées suivant deux couronnes concentriques sur un fond de cuve 2 ; chacune des têtes de remplissage 1 est reliée à un réservoir de liquide 3 par un élévateur à bulles 4 qui se termine par une aiguille 5 fixée sur un plot de prélèvement 6. Le fond de cuve 2 est une enceinte très polluée, surmontée d'un couvercle ou bouchon 7 qui l'isole de l'extérieur mais est cependant percé et occupé à l'endroit du perçage par un porte-outil 8. Le bouchon 7 tourne autour d'un axe non représenté qui coïncide avec celui des couronnes de têtes de remplissage 1.

Ce porte-outil 8 comprend une conduite de raccord 9 à un réseau de conduites de transport par voie pneumatique dont on a représenté une extrémité 10, mobile verticalement jusqu'à s'aboucher à la conduite de raccord 9. Le porte-outil 8 comprend encore un arbre médian 11 tournant autour de son axe pour amener un fourreau 12, situé d'abord sous la conduite de raccord 9 ou au-dessus d'une tête de remplissage 1 d'une des couronnes, au-dessus d'un endroit de l'autre couronne de têtes de remplissage 1 si nécessaire. L'arbre médian 11 traverse le porte-outil 8 de part en part et s'étend au-dessous de lui.

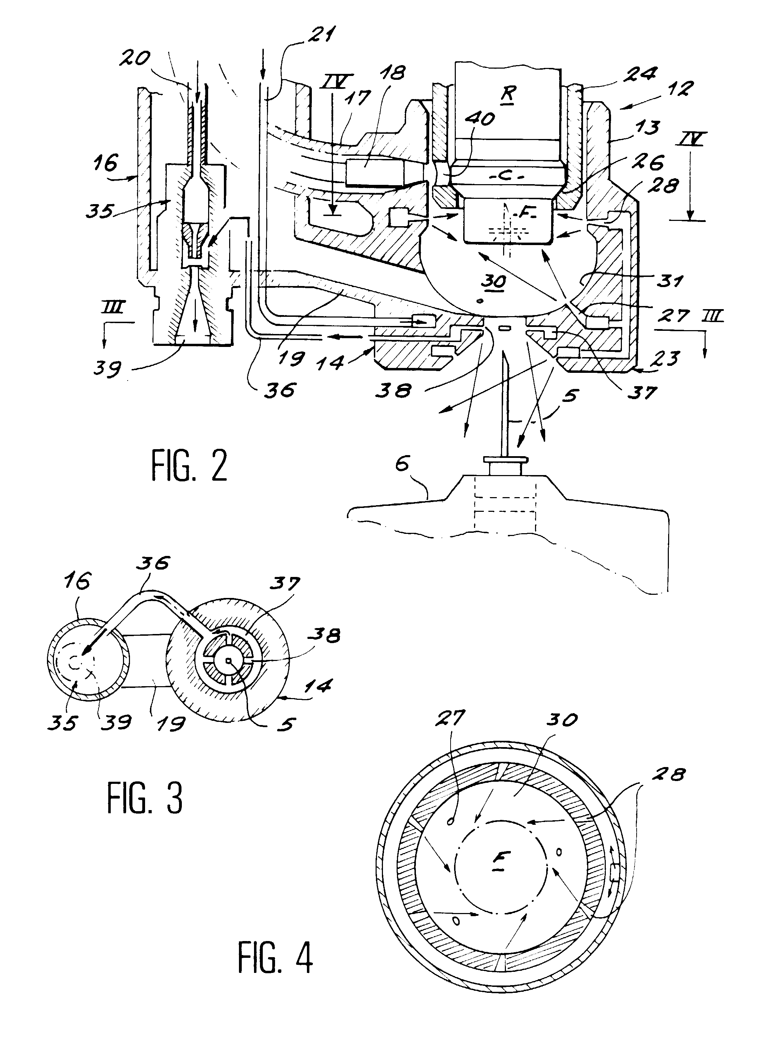

Le fourreau 12, qui est plus visible à la figure 2, comprend en particulier une partie cylindrique 13 supérieure jointe à un fond 14 en forme de coupelle et dont la paroi est percée juste devant une des aiguilles 5. Le perçage est désigné par 15.

Le fourreau 12 est relié à un piston creux 16 par un conduit de fibre optique 17, qui s'étend dans le piston creux 16 puis traverse sa paroi pour aboutir dans la partie cylindrique 13 (la fibre optique est désignée par 18), et par un raccordement d'air 19 qui débouche de côté dans le fond 14. Le raccordement d'air 19 communique dans l'intérieur du piston creux 16, qui peut être rempli d'air par des moyens non représentés situés au-dessus du porte-outil 8.

En référence à la figure 1, le piston creux 16 contient encore une canalisation d'air 20 et une canalisation d'eau 21 et coulisse dans des paliers 22 axialement à l'intérieur de l'arbre 11 pour abaisser le fourreau 12 de la position représentée à celle qui est indiquée en traits mixtes, où une collerette inférieure 23 érigée sous la face inférieure du fond 14 se pose sur le plot 6 autour de l'aiguille 5 choisie, qui entre dans le fond 14 par le perçage 15.

Le porte-outil 8 est lui-même mobile dans la direction de coulisse du piston creux 16, et il enfonce alors une nacelle 24 de forme cylindrique et partiellement contenue dans le fourreau 12, vers le fond de celui-ci. Le porte-outil 8, porteur de l'arbre médian 11, et le piston creux 16 sont donc mobiles indépendamment en translation, mais l'arbre médian 11 et le piston creux 16 sont solidaires en rotation à cause de l'engagement de la nacelle 24 dans le fourreau 12 et de la fixation de la nacelle 24 à un plateau 25 que l'arbre médian 11 meut. Un récipient R, appelé cruchon-curseur par la déposante, retenu et posé dans la nacelle 24 et dont le fond F dépasse de celle-ci par un trou 26 qui traverse la face inférieure de la nacelle 24, est ainsi déplacé et mis au-dessus d'une tête de prélèvement 1, choisie en fonction de la rotation du bouchon toutnant 7, puis abaissé sur l'aiguille 5 de la tête de prélèvement 1, qui perce finalement le fond F. Plus précisément, la rotation du bouchon tournant 7 amène le récipient R à portée d'une tête de prélèvement 1 de chacune des couronnes, et le choix entre ces deux têtes est accompli par une rotation de l'arbre médian 11, dont l'axe est à mi-distance des deux couronnes et sur lequel le récipient R est excentré. Une pression d'air sur le sommet du récipient R peut s'ajouter au poid du récipient R pour imposer le perçage.

Les différentes pièces mobiles sont mues par des moteurs électriques ou des moyens pneumatiques et des transmissions qui ne sont pas représentés car ils ne font l'objet d'aucun détail original et sont présents sur l'installation existante et connue. Les canalisations sont raccordées à des canalisations fixes d'alimentation en eau ou en air par l'intermédiaire de tuyaux souples au-dessus du porte-outil 8 et qui ne sont pas non plus représentés.

En référence à la figure 2, la canalisation d'eau 21 se divise hors du piston creux 16, à l'intérieur du fond 14 du fourreau 12 ou devant lui, pour alimenter des buses d'aspersion placées dans le fond 14 du fourreau 12 : on en compte dix dans la réalisation provoquée ici, soit trois buses 27 d'aspersion du récipient R, six buses de barrage 28 et une buse 29 d'aspersion de l'aiguille 5. Les trois buses 27 d'aspersion du récipient débouchent dans le creux 30 délimité par une face interne 31 de la paroi du fond 14 et près du bas de ce creux 30, non loin du perçage 15, et elles sont orientées (voir aussi la figure 4) suffisamment obliquement pour projeter l'eau vers le fond F du récipient R, et avec une incidence latérale suffisante pour que, quand l'eau a rebondi sur le fond F du récipient R vers la face interne 31, un tourbillon soit produit sur toute la surface de la face interne 31 qui est ainsi complètement arrosée et lavée : les gouttelettes de liquide contaminant originaires de l'aiguille 5 après un jaillissement intempestif éventuel s'écoulent par le perçage 15 et coulent ensuite le long du plot 6 puis dans un égout non représenté situé au-dessous du fond de cuve 2. Cet égout peut être un simple collecteur de ruissellement par gravité, et sa conception ne nécessite pas de finesses particulières car la chambre de remplissage située sous le bouchon tournant 7 peut rester polluée sans inconvénient.

La face interne 31 est courbe et lisse, sans changement brusque de pente qui produirait des recoins dans lesquels les gouttelettes auraient tendance à stagner.

Les six buses de barrage 28 débouchent tout au sommet du creux 30, presque à hauteur de la partie cylindrique 13 et émettent des jets à faible débit et à faible pression, jets horizontaux concourants et tangents au fond F qui achèvent de laver ce fond F, en particulier sur la portion cylindrique adjacente à sa face plane d'extrémité que l'aiguille 5 pique. Les jets issus des buses de barrage 28 ont également pour fonction de couper les portions de jets originaires des buses 27 d'aspersion du récipient R qui s'élèveraient exagérément et pourraient projeter des gouttelettes éventuellement contaminées sur des parties plus élevées du récipient R. Les buses de barrage 28 sont situées au-dessus des buses 27 d'aspersion du récipient R et leurs jets peuvent aussi être obliques latéralement pour renforcer le tourbillon sur la face interne 31.

La buse 29 d'aspersion de l'aiguille est logée dans la collerette 23 en direction de l'aiguille 5 et sert à nettoyer celle-ci. Elle est unique mais on pourrait en disposer plusieurs autour de l'aiguille 5. Comme cette buse 21 est mise en service après les autres, quand l'eau de lavage du creux 30 et du fond F a coulé, elle nécessite un réseau d'alimentation particulier, ce qui explique qu'on puisse être tenté de l'omettre du dispositif. Elle n'est d'ailleurs pas indispensable et relève plutôt d'une précaution supplémentaire.

L'installation comprend également un système de séchage, bien visible sur les figures 2 et 3, qui comprend une trompe d'aspiration 35 en bas de la canalisation d'air comprimé 20 pour y créer localement un rétrécissement de section et une dépression ; un tuyau 36 débouche au col de la trompe d'aspiration 35 et aboutit dans une gorge 37 établie derrière le sommet de la face interne 31 et que seuls quatre orifices 38 relient au creux 30. Ces orifices 38 sont donc des orifices d'aspiration par lesquels l'eau résiduelle au lavage est aspirée de la face interne 31 et du fond F du récipient R, puis éjectée par l'orifice 39 de la canalisation d'air comprimé 20. Le dispositif d'aspiration par combinaison d'un courant d'air et d'un moyen statique de création de dépression est astucieux car l'air peut être distrait de l'alimentation en air des conduites de transport presque sans complication de l'installation.

Le récipient R peut être renvoyé dans le réseau 10 après le séchage. On envoie pour cela de l'air dans le piston creux 16 et dans le raccordement d'air 19. La fibre optique 18 permet de vérifier qu'un récipient R est au fond de la nacelle 24.

La nacelle 24 est percée d'une fenêtre 40 qui dévoile normalement une portion d'une collerette C du récipient R, située juste au-dessus du fond F et dont l'utilité est de guider le récipient R dans les conduites d'air comprimé et accessoirement de retenir le récipient R dans la nacelle 24 en l'empêchant de tomber par le trou 26. La collerette C est repérable par la fibre optique 18 grâce à sa couleur ou à une autre caractéristique d'identification.

Le lavage illustré sur les figures 2 et 4 se déroule quand le fourreau 12 et la nacelle 24 ont été relevés de l'aiguille 5 de perçage et sont revenus à la position de la figure 1 après le remplissage du récipient R.

Le séchage est fait après un retour du fourreau 12 sur le plot 6, la nacelle 24 restant soulevée pour écarter le fond F de l'aiguille 5, afin de boucher le perçage au bas du creux 30 et de permettre de renvoyer le récipient R vers le haut et dans le réseau de transfert pneumatique par de l'air fourni par le piston creux 16.

## Revendications

1. Dispositif de remplissage d'un récipient clos (R), comprenant : un élévateur de liquide (4) de remplissage terminé par une aiguille (5) de perçage d'un fond (F) du récipient ; un fourreau (12) ayant un fond (14) constitué par une paroi percée (15) devant l'aiguille (5), mobile vers l'aiguille et se raccordant à un réseau de conduits (9, 10) de transport du récipient par voie pneumatique ; et une nacelle (24) située dans le fourreau et agencée pour suspendre le récipient (R) un peu au-dessus du fond (14) du fourreau ; caractérisé en ce que le fourreau (12) est équipé de buses (27, 28) d'aspersion de liquide de lavage vers le fond (F) du récipient suspendu dans la nacelle et vers la face interne (31) de la paroi du fond du fourreau.

2. Dispositif de remplissage suivant la revendication 1, caractérisé en ce que certaines des buses (27) sont orientées de façon à asperger le fond du récipient obliquement et à produire un rebondissement du liquide de lavage du fond du récipient vers la face interne du fond du fourreau.

3. Dispositif de remplissage suivant la revendications 2, caractérisé en ce que certaines des buses (28) sont orientées tangentiellement au fond du récipient, à hauteur du fond (F) du récipient et d'une portion adjacente du récipient.

4. Dispositif de remplissage suivant l'une quelconque des revendications 1 à 3, caractérisé en ce que certaines des buses (27, 28) sont orientées de façon à asperger la face interne (31) du fond du fourreau obliquement et à y produire un tourbillon du liquide de lavage.

5. Dispositif de remplissage suivant l'une quelconque des revendications 1 à 4, caractérisé en ce que le fourreau est équipé d'au moins une buse (29) d'aspersion de liquide vers l'aiguille.

6. Dispositif de remplissage suivant l'une quelconque des revendications 1 à 5, caractérisé en ce que le fourreau est équipé de moyens de séchage (35 à 38) du fond (F) du récipient (R) et de la face interne (31) de la paroi du fond (14) du fourreau (12).

7. Dispositif de remplissage suivant la revendication 6, caractérisé en ce que les moyens de séchage consistent en des moyens d'aspiration animés par un circuit d'air comprimé (20) muni d'un moyen statique de création de dépression (35).

## Patentansprüche

1. Vorrichtung zum Füllen eines geschlossenen Behälters (R), umfassend: einen mit einer Nadel (5) zum Durchstechen des Bodens (14) des Behälters (F) endenden Flüssigkeitsfüllungsheber (4), eine Stulpe (12) mit einem Boden (14), gebildet durch eine vor der Nadel (5) durchlochte Wand (15); beweglich in Richtung Nadel und angeschlossen an ein Netz von Leitungen (9, 10) zur Beförderung der Behälter auf pneumatischem Wege; und einen Korb (24), in der Stulpe befindlich und angebracht, um den Behälter (R) etwas über dem Boden (14) der Stulpe zu halten;
**dadurch gekennzeichnet**,
daß die Stulpe (12) mit Düsen (27, 28) ausgestattet ist, um den Boden (F) des durch die Stulpe gehaltenen Behälters und die Innenseite (31) des Stulpenbodens mit Reinigungsflüssigkeit zu bespritzen.

2. Vorrichtung nach Anspruch 1, dadurch gekennzeichnet, daß einige der Düsen (27) so ausgerichtet sind, daß sie den Boden des Behälters schräg bespritzen und ein Zurückspritzen der Reinigungsflüssigkeit vom Boden des Behälters in Richtung Innenseite des Stulpenbodens verursachen.

3. Vorrichtung nach Anspruch 2, dadurch gekennzeichnet, daß einige der Düsen (28) tangential zum Behälterboden ausgerichtet sind, in Höhe des Bodens (F) des Behälters und eines angrenzenden Teils des Behälters.

4. Vorrichtung nach einem der Ansprüche 1 bis 3, dadurch gekennzeichnet, daß einige der Düsen (27, 28) so ausgerichtet sind, daß sie die Innenseite (31) des Bodens der Stulpe schräg bespritzen und dort einen Reinigungsflüssigkeitswirbel bilden.

5. Vorrichtung nach einem der Ansprüche 1 bis 4, dadurch gekennzeichnet, daß die Stulpe mit wenigstens einer Düse (29) zum Bespritzen der Nadel mit Reinigungsflüssigkeit versehen ist.

6. Vorrichtung nach einem der Ansprüche 1 bis 5, dadurch gekennzeichnet, daß die Stulpe Trocknungseinrichtungen (35 bis 38) des Bodens (F) des Behälters (R) und der Innenseite (31) der Bodenwand (14) der Stulpe (12) umfaßt.

7. Vorrichtung nach Anspruch 6, dadurch gekennzeichnet, daß die Trocknungseinrichtungen gebildet werden durch ein Saugsystem, das eine Druckluftleitung (20) und eine statische Unterdruckerzeugungseinrichtung (35) umfaßt.

## Claims

1. Device for filling a receptacle (R) including: a filling liquid elevator (4) ended by a needle (5) for piercing a bottom (F) of the receptacle; a sheath (12) having a bottom (14) constituted by a wall (15) pierced in front of the needle (5) and mobile towards the needle and being connected to a network of pipes (9, 10) for carrying the receptacle by pneumatic means; and a boat (23) situated in the sheath and disposed so as to suspend the receptacle (R) slightly above the bottom (14) of the sheath, characterized in that the sheath (12) is equipped with nozzles (27, 28) for sprinkling washing liquid towards the bottom (F) of the receptacle suspended in the boat and towards the internal face (31) of the wall of the bottom of the sheath.

2. Filling device according to claim 1, characterized in that some of the nozzles (27) are oriented in such a way so as to sprinkle the bottom of the receptacle obliquely and produce a rebounding of the washing liquid from the bottom of the receptacle towards the internal face of the bottom of the sheath.

3. Filling device according to claim 2, characterized in that some of the nozzles (28) are oriented tangentially to the bottom of the receptacle, the height of the bottom (F) of the receptacle and an adjacent portion of the receptacle.

4. Filling device according to any one of the claims 1 to 3, characterized in that some of the nozzles (27, 28) are oriented in such a way so as to sprinkle the internal face (31) of the bottom of the sheath obliquely and produce there a vortex of the washing liquid.

5. Filling device according to any one of the claims 1 to 4, characterized in that the sheath is equipped with at least one nozzle (29) for sprinkling liquid towards the needle.

6. Filling device according to any one of the claims 1 to 5, characterized in that the sheath is equipped with means (35 to 38) for drying the bottom (F) of the receptacle (R) and internal face (31) of the wall of the bottom (14) of the sheath (12).

7. Filling device according to claim 6, characterized in that the drying means consists of suction means driven by a compressed air circuit (20) provided with a static partial depression creation device (35).
